# EUROPEAN PATENT SPECIFICATION

(11) **EP 3 616 741 B1**
(45) Date of publication and mention of the grant of the patent: **29.04.2026**
(21) Application number: 18790541.9
(22) Date of filing: 27.04.2018
(51) Int. Cl.: A61M 25/06, A61M 25/00

(54) **AIR REMOVAL MECHANISM AND INTRODUCER SHEATH**
LUFTENTFERNUNGSMECHANISMUS UND EINFÜHRSCHLEUSE
MÉCANISME D'ÉLIMINATION D'AIR ET GAINE D'INTRODUCTION

(30) Priority: 28.04.2017 JP 2017089434
(43) Date of publication of application: 04.03.2020
(73) Proprietor: Terumo Kabushiki Kaisha, Tokyo 151-0072 (JP)
(72) Inventor: MORI, Takayuki, Fujinomiya-shi Shizuoka 418-0015 (JP); HARADA, Kinya, Fujinomiya-shi Shizuoka 418-0015 (JP); KARINO, Wataru, Tokyo 163-1450 (JP); SHIMIZU, Katsuhiko, Ashigarakami-gun Kanagawa 259-0151 (JP); KUWANO, Youichirou, Ashigarakami-gun Kanagawa 259-0151 (JP)
(74) Representative: Casalonga
(86) International application number: PCT/JP2018/017232
(87) International publication number: WO 2018/199297

(56) References cited:
- JP-A- 2007 089 607
- JP-A- 2016 152 841
- JP-A- 2016 152 841
- US-A- 5 507 732
- US-A- 5 507 732
- US-A1- 2013 331 692
- US-A1- 2016 375 222
- US-B1- 7 335 182

## Description

### Technical Field

The present invention relates to an air removal mechanism for use with an introducer sheath which are applicable to an introducer sheath.
In particular, the present invention relates to an air removal mechanism according to the preamble of claim 1, such as it is e.g. known from US 7 335 182 B1.
Other related prior art showing mechanism for air removal from an introducer sheath are e.g. shown in
- JP 2016 15284 A
- US 2016/375222 A1
- US 5 507 732 A
- US 2013/331692 A1

### Background Art

In the related art, an introducer sheath having a lumen and an introducer including a dilator used by being inserted into the sheath are known (for example, refer to JP-A-8-71161) . After the introducer is inserted into a blood vessel along a thin guide wire (first guide wire), the dilator is pulled out of the introducer sheath together with the first guide wire. Then, the introducer sheath is inserted together with a thick guide wire (second guide wire) , and a catheter is inserted along the second guide wire, thereby performing desired treatment.

### Summary of Invention

Incidentally, when another medical device such as the catheter is inserted into the introducer sheath, or when a medical device is removed from the introducer sheath, air may be mixed into the introducer sheath in some cases. There is a possibility that the air mixed into the introducer sheath may enter a living body.

The present invention is made in view of this problem, and an object thereof is to provide an air removal mechanism to be used with an introducer sheath which can prevent air from being mixed into the introducer sheath when a medical device is inserted or removed.

According to the present invention, in order to achieve the above-described object, there is provided an air removal mechanism according to independent claim 1. The dependent claims relate to advantageous embodiments.

According to the air removal mechanism of the present invention including the above-described configuration, if the liquid is injected via the injection path disposed in the body into the hollow portion of the air removal mechanism located in the sheath proximal portion having the valve body facing upward, air existing above the valve body (air inside the hollow portion) is extruded by the liquid. Therefore, the air existing above the valve body is removed. Therefore, when another medical device such as a catheter is inserted into the introducer sheath, or when the medical device is removed from the introducer sheath, the air can be prevented from being mixed into the introducer sheath.

In the injection path, at least an end portion flow path on the outlet side may be inclined with respect to an axis of the body.

In this manner, according to a simple configuration, the injection path on which the outlet is open toward the valve body can be disposed inside the body.

The outlet of the injection path is open toward a position different from a center of the valve body.

According to this configuration, the liquid is likely to reach an outer peripheral side of the valve body. Therefore, the air existing above the valve body can be effectively removed.

The outlet of the injection path may be formed so that the liquid reaches the valve body in a tangential direction of the valve body when viewed in an axial direction of the valve body.

According to this configuration, when the liquid flowing out of the outlet of the injection path reaches the valve body, a swirling flow of the liquid is formed above the valve body. Therefore, the air existing above the valve body can be more effectively removed.

The air removal mechanism may further include a side port which protrudes from an outer peripheral portion of the body, in which an inlet of the injection path is formed, and to which a liquid injection device is connectable.

According to this configuration, the liquid injection device is connected to the side port, and the liquid is discharged from the liquid injection device. In this manner, the liquid can be easily injected into the hollow portion via the injection path.

The side port may be inclined with respect to an axis of the body.

According to this configuration, when in use, the side port or the liquid injection device is less likely to cause trouble.

The body may be configured to be separated from the sheath proximal portion.

According to this configuration, a configuration of the introducer sheath does not need to be greatly changed from the introducer sheath in the related art, and the air can be prevented from being mixed into the introducer sheath.

The air removal mechanism may further include a seal member held by the body and sealing a portion between the body and the sheath proximal portion in a liquid-tight manner.

According to this configuration, the liquid injected into the hollow portion is prevented from leaking out of a portion between the body and the sheath proximal portion. Therefore, an air mixing prevention function can be properly fulfilled when the medical device is inserted or removed.

The body may be disposed integrally with the sheath proximal portion.

According to this configuration, the hollow portion of the air removal mechanism is filled with the liquid before a dilator is inserted into the introducer sheath. In this manner, when the dilator is removed from the introducer sheath, the air can be prevented from being mixed into the sheath.

The body may be transparent.

According to this configuration, it is possible to visibly confirm a decrease in the liquid or a stored liquid level inside the body which is caused by the insertion and removal of the medical device. Therefore, the liquid can be injected from the injection path before the liquid existing above the valve body runs short.

The side port may be provided with another valve body having higher liquid-tightness than the valve body disposed inside the sheath proximal portion.

According to this configuration, when the liquid is injected from the side port, the liquid injection device can be fixed to the valve body without any gap. When the liquid injection device is separated from the side port, the valve body disposed in the side port has higher liquid-tightness than the valve body disposed inside the sheath proximal portion. Therefore, the liquid-tightness can be maintained inside the side port.

A flow path cross-sectional area of the injection path may be smaller than a flow path cross-sectional area of the hollow portion.

According to this configuration, a flow velocity of the liquid is allowed to increase. Therefore, the air inside the injection path or the body can be more effectively removed.

An inner diameter and an outer diameter of a proximal portion of the body may be respectively larger than an inner diameter and an outer diameter of a distal portion of the body.

According to this configuration, a difference between the outer diameters of the proximal portion and the distal portion of the body functions as a scale for measuring a liquid level. In this manner, if the liquid reaches the distal portion of the body, the distal portion serves as a marker for injecting the liquid. Therefore, it is possible to inject the liquid from the injection path before the liquid existing above the valve body runs short. In addition, the inner diameter of the proximal portion is larger than the inner diameter of the distal portion in the body, which makes it easy to insert the medical device.

The air removal mechanism according to the present invention, may be used with an introducer sheath having a valve body disposed inside a sheath proximal portion. The introducer sheath includes a sheath main body portion, and an air removal mechanism according to the invention may be integrally disposed in the sheath proximal portion. The air removal mechanism includes a body having a hollow portion capable of storing a liquid. A medical device to be inserted into the introducer sheath is insertable via the hollow portion. The body has an injection path communicating with the hollow portion and having an outlet which is open toward the valve body.

According to the air removal mechanism of the present invention, the air can be prevented from being mixed into the introducer sheath when the medical device is inserted or removed.

### Brief Description of Drawings

[Fig. 1] Fig. 1 is a schematic view of an introducer including an air removal mechanism according to a first embodiment of the present invention.
[Fig. 2] Fig. 2 is a view illustrating a state where a dilator is inserted into a sheath illustrated in Fig. 1.
[Fig. 3] Fig. 3 is a sectional view illustrating a state where the air removal mechanism is attached to a proximal portion of the sheath illustrated in Fig. 1.
[Fig. 4] Fig. 4A is a plan view of the air removal mechanism of an illustrative example not falling under the scope of the claims. Fig. 4B is a plan view of an air removal mechanism according to the invention.
[Fig. 5] Fig. 5 is a first view for describing an operation of the introducer illustrated in Fig. 1.
[Fig. 6] Fig. 6 is a second view for describing an operation of the introducer illustrated in Fig. 1.
[Fig. 7] Fig. 7 is a third view for describing an operation of the introducer illustrated in Fig. 1.
[Fig. 8] Fig. 8 is a fourth view for describing an operation of the introducer illustrated in Fig. 1.
[Fig. 9] Fig. 9 is a fifth view for describing an operation of the introducer illustrated in Fig. 1.
[Fig. 10] Fig. 10 is a schematic view of an introducer including an air removal mechanism according to a second embodiment of the present invention.
[Fig. 11] Fig. 11 is a sectional view of a proximal portion of a sheath illustrated in Fig. 10.
[Fig. 12] Fig. 12 is a first view for describing an operation of the introducer illustrated in Fig. 10.
[Fig. 13] Fig. 13 is a second view for describing an operation of the introducer illustrated in Fig. 10.
[Fig. 14] Fig. 14 is a third view for describing an operation of the introducer illustrated in Fig. 10.
[Fig. 15] Fig. 15 is a fourth view for describing an operation of the introducer illustrated in Fig. 10.
[Fig. 16] Fig. 16 is a fifth view for describing an operation of the introducer illustrated in Fig. 10.
[Fig. 17] Fig. 17 is a sixth view for describing an operation of the introducer illustrated in Fig. 10.
[Fig. 18] Fig. 18 is a schematic view of an introducer including an air removal mechanism according to a third embodiment of the present invention.
[Fig. 19] Fig. 19 is a sectional view illustrating a state where the air removal mechanism is attached to a proximal portion of a sheath illustrated in Fig. 18.
[Fig. 20] Fig. 20 is a first view for describing an operation of the introducer illustrated in Fig. 18.
[Fig. 21] Fig. 21 is a second view for describing an operation of the introducer illustrated in Fig. 18.
[Fig. 22] Fig. 22 is a third view for describing an operation of the introducer illustrated in Fig. 18.
[Fig. 23] Fig. 23 is a fourth view for describing an operation of the introducer illustrated in Fig. 18.
[Fig. 24] Fig. 24 is a fifth view for describing an operation of the introducer illustrated in Fig. 18.
[Fig. 25] Fig. 25 is a sixth view for describing an operation of the introducer illustrated in Fig. 18.
[Fig. 26] Fig. 26 is a schematic sectional view of the air removal mechanism according to the third embodiment of the present invention.
[Fig. 27] Fig. 27A is a sectional view of a connector before a liquid injection device is connected to the connector. Fig. 27B is a sectional view of the connector in a state where the liquid injection device is connected to the connector.

### Description of Embodiments

Hereinafter, a plurality of preferred embodiments relating to an air removal mechanism according to the present invention will be described with reference to the accompanying drawings. In second and third embodiments, the same reference numerals will be given to elements which are the same as or similar to elements according to a first embodiment, and detailed description thereof will be omitted.

### [First Embodiment]

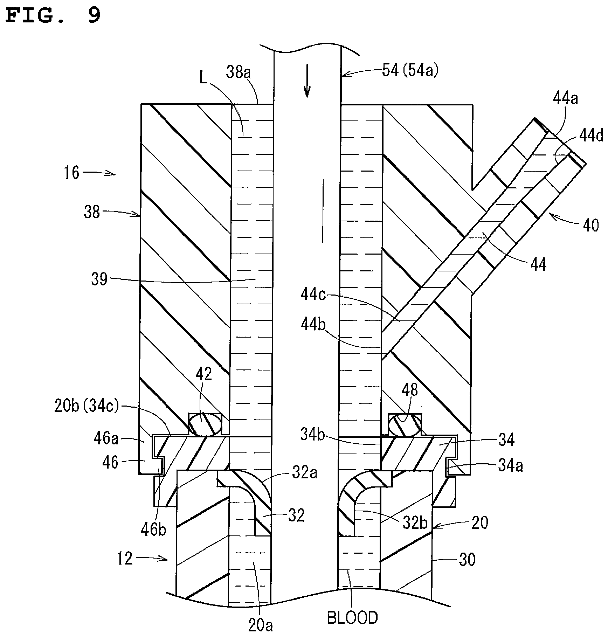

An introducer 10 illustrated inFig. 1 is usedbybeing inserted into a living body (blood vessel) so that a medical device 54 (refer to Fig. 9) such as a catheter is delivered to a desired position inside the living body. For example, the medical device 54 includes a diagnostic catheter, a guiding catheter, a balloon catheter, a stent delivery catheter, an ablation catheter, and a guide wire.

The introducer 10 includes an introducer sheath 12 (hereinafter, simply referred to as a sheath 12) having a lumen 18a, a dilator 14 used by being inserted into the lumen 18a of the sheath 12, and an air removal mechanism 16 according to the first embodiment which is used in combination with the sheath 12.

The sheath 12 has an elongated sheath shaft 18 configuring a sheath main body and a sheath hub 20 connected to a proximal end of the sheath shaft 18. The dilator 14 has an elongated dilator shaft 22 configuring a dilator main body and a dilator hub 24 connected to a proximal end of the dilator shaft 22.

The lumen 18a is formed in the sheath shaft 18. The sheath shaft 18 is elastic (flexible) so as to easily follow a meandering blood vessel, and is properly rigid so as not to be bent inside the blood vessel. Configuration materials of the sheath shaft 18 include polyolefin (for example, polyethylene, polypropylene, polybutene, ethylene-propylene copolymer, ethylene-vinyl acetate copolymer, ionomer, or a mixture of the above-described two or more materials) , polymer materials such as polyvinyl chloride, polyamide, polyamide elastomer, polyester, polyester elastomer, polyurethane, polyurethane elastomer, polyimide, fluorine resin, or a mixture thereof, or the above-described two or more polymer materials. The configuration material of the sheath shaft 18 also includes a contrast agent such as a filler. In addition, as a reinforcement body, the sheath shaft 18 also includes a metal wire such as a stainless steel wire.

The sheath hub 20 is a member configuring a sheath proximal portion, has a lumen 20a communicating with the lumen 18a formed inside the sheath shaft 18, and the dilator shaft 22 can be inserted. The sheath hub 20 is formed of a hard material (hard resin material). One end of a tube 26 is connected to a connection port 21 disposed in the sheath hub 20. A stopcock 28 is disposed in the other end of the tube 26. A liquid for medical treatment (contrast agent, physiological salt solution, or drug solution) can be supplied into the sheath hub 20 via the stopcock 28 and the tube 26, and can be injected into a living body via the sheath shaft 18.

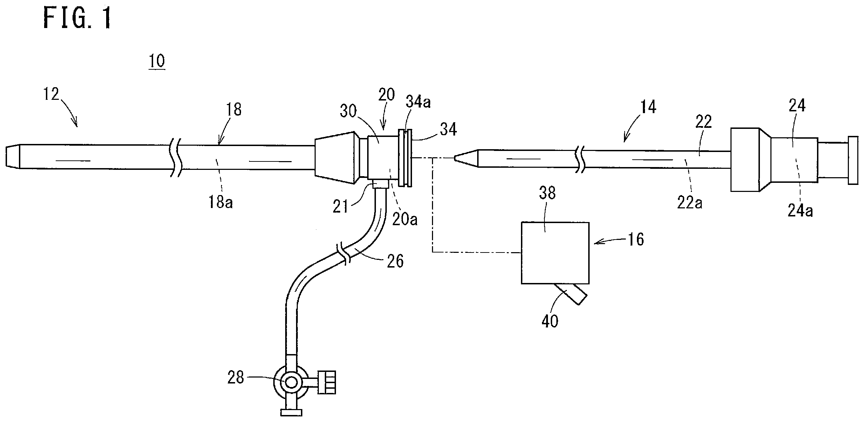

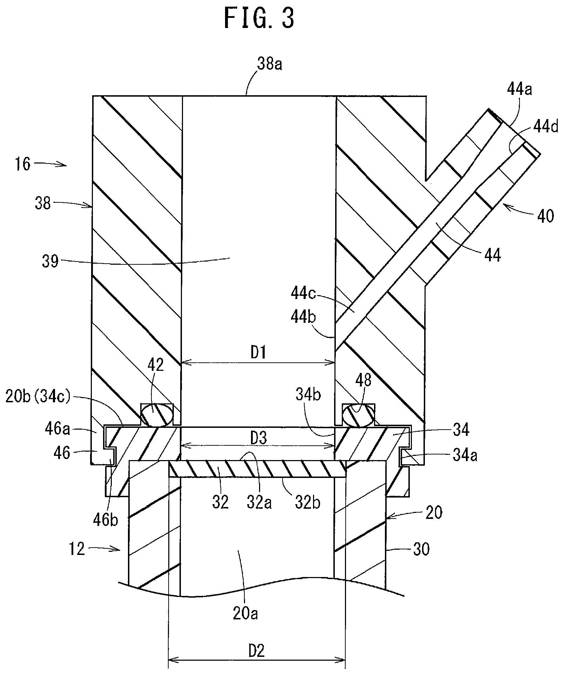

As illustrated in Fig. 3, the sheath hub 20 has a housing 30 surrounding the lumen 20a, a valve body 32 located in a proximal portion of the housing 30, and a fixing member 34 for fixing the valve body 32 to the housing 30. In Fig. 3, the air removal mechanism 16 is mounted on (connected to) a proximal portion of the sheath hub 20. The housing 30 is formed as a hollow cylindrical body. A connection port (refer to Fig. 1) is disposed in the housing 30.

The valve body 32 is a plate-shaped member (disc-shaped member) formed of an elastic material such as a rubber material and an elastomer material, and prevents the liquid such as blood from leaking out of the sheath hub 20. Although not illustrated in detail, one surface 32a and the other surface 32b of the valve body 32 have two slits which intersect each other when viewed in the axial direction of the valve body 32. when the dilator 14 or a catheter is not inserted into the sheath 12, the two slits are closed, and the lumen 20a inside the sheath hub 20 is sealed in a liquid-tight manner.

The fixing member 34 has a circular opening portion 34b through which the valve body 32 is exposed in a proximal end direction. An inner peripheral portion of the fixing member 34 is attached to an outer peripheral portion of the valve body 32. The fixing member 34 is fixed to the proximal portion of the housing 30 by means of welding or by using an adhesive, for example. An annular groove 34a is formed on an outer peripheral surface of the fixing member 34.

In Fig. 1, the dilator shaft 22 is elastic (flexible) so as to easily follow the meandering blood vessel, and is properly rigid so as not to be bent inside the blood vessel. The dilator shaft 22 is a tubular member having a lumen 22a. An outer diameter of the dilator shaft 22 is smaller than an outer diameter of the sheath 12. A configuration material of the dilator shaft 22 may be the same as a configuration material of the sheath shaft 18.

The dilator hub 24 has a lumen 24a communicating with the lumen 22a formed inside the dilator shaft 22. The dilator hub 24 is attachable to and detachable from the proximal portion of the sheath hub 20, and is formed of a hard material (hard resin material) . A distal inner periphery of the dilator hub 24 has an engagement claw which can engage with the annular groove 34a disposed in a proximal outer periphery of the sheath hub 20. Therefore, the dilator hub 24 can be externally fitted to the proximal portion of the sheath hub 20. The dilator hub 24 may be configured to be internally fitted to the proximal portion of the sheath hub 20.

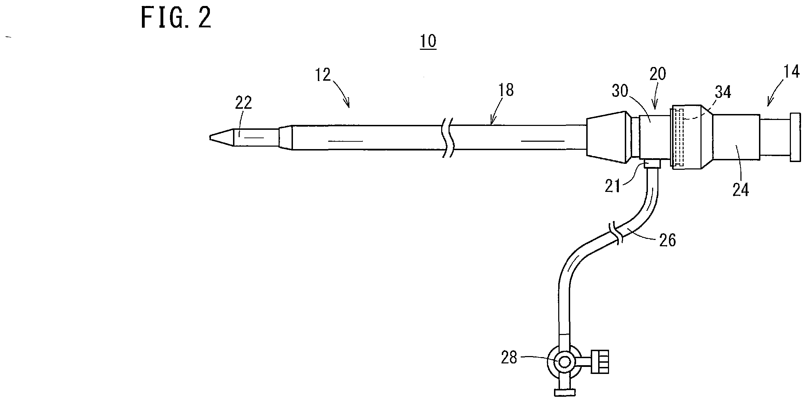

As illustrated in Fig. 2, in a state where the dilator 14 is inserted into the sheath 12 (state where the sheath shaft 18 is inserted into the dilator shaft 22 and the dilator hub 24 is connected to the sheath hub 20), a distal portion of the dilator shaft 22 protrudes as much as a predetermined length from a distal opening of the sheath shaft 18. In an initial state of the introducer 10, therefore, the sheath 12 and the dilator 14 are in a combined state (assembled state).

In Fig. 1, the air removal mechanism 16 is configured to be mountable on the proximal portion of the sheath hub 20. In the present embodiment, the air removal mechanism 16 is attachable to and detachable from the sheath hub 20 (air removal mechanism 16 is removable after being mounted on the sheath hub 20). The air removal mechanism 16 may be configured so as not to be removable after being mounted once on the sheath hub 20.

In Fig. 3, the air removal mechanism 16 is mounted on (connected to) the proximal portion of the sheath hub 20. The air removal mechanism 16 includes a body 38 having a hollow portion 39 capable of storing a liquid L, a side port 40 protruding from an outer peripheral portion of the body 38, and a seal member 42 for sealing a portion between the body 38 and the sheath hub 20 in a liquid-tight manner.

The hollow portion 39 is formed to face the valve body 32, and the medical device 54 (refer to Fig. 9) to be inserted in the sheath 12 is configured to be insertable. The hollow portion 39 is formed in a straight line shape along the axis of the sheath hub 20, and a cross-sectional shape perpendicular to the axis of the body 38 is a circular shape. In Fig. 3, a diameter D1 of the hollow portion 39 is constant along the axial direction of the body 38, and the diameter D1 of at least the distal portion of the hollow portion 39 is smaller than an outer diameter D2 of the valve body 32.

The diameter D1 of the hollow portion 39 may be changed along the axial direction of the body 38. For example, the diameter D1 of the hollow portion 39 may increase in a proximal end direction. The axis (central axis) of the hollowportion 39 is located coaxially with the axis (central axis) of the valve body 32. The diameter D1 of the hollow portion 39 may be the same as a diameter D3 of the circular opening portion 34b of the fixing member 34, and may be smaller or larger than the diameter D3. The body 38 is transparent.

The body 38 has an injection path 44 communicating with the hollow portion 39 and having an outlet 44b which is open toward the valve body 32 (portion exposed through the circular opening portion 34b within one surface 32a of the valve body 32). In other words, the valve body 32 is located on an extension line of the injection path 44. Specifically, the injection path 44 is disposed in the body 38 and the side port 40. An inlet 44a of the injection path 44 is open on a protruding side end surface of the side port 40. The outlet 44b of the injection path 44 is open on an inner peripheral surface of the body 38. In the present embodiment, the outlet 44bof the injection path 44 is open toward a position different from a center of the valve body 32 in the valve body 32.

In the injection path 44, at least an end portion flow path 44c on the outlet 44b side is inclined with respect to the axis of the body 38. In the injection path 44, a range other than the end portion flow path 44c on the outlet 44b side may have a portion which is not inclined with respect to the axis of the body 38. The diameter of the injection path 44 is smaller than the diameter of the hollow portion 39. Therefore, a flow path cross-sectional area of the injection path 44 is smaller than a flow path cross-sectional area of the hollow portion 39.

The distal portion of the body 38 has a connection-purpose engagement portion 46 which can engage with the sheath hub 20. In the present embodiment, the connection-purpose engagement portion 46 has an elastic piece 46a protruding in a distal end direction from the distal portion of the body 38. A claw portion 46b which can engage with the annular groove 34a disposed in the sheath hub 20 (fixing member 34) is disposed inside the distal end of the elastic piece 46a. A plurality of the claw portions 46b are arranged at an interval in a circumferential direction.

As a connection structure between the sheath hub 20 and the body 38, an annular groove may be disposed in the body 38 instead of the above-described configuration. A claw portion which can engage with the annular groove may be disposed in the sheath hub 20. As the connection structure between the sheath hub 20 and the body 38, a screwing structure may be provided instead of the above-described configuration.

The side port 40 is configured to be connectable to the liquid injection device. For example, the liquid injection device includes a syringe having a nozzle in a distal end. The inner peripheral surface of an end portion (end portion on the inlet 44a side) of the side port 40 is formed as a lure taper 44d whose diameter decreases toward the outlet 44b side so that a discharge portion (nozzle of the syringe) of the liquid injection device can be connected (fitted) thereto in a liquid-tight manner.

In the first embodiment, the side port 40 is inclined with respect to the axis of the body 38. Specifically, the side port 40 is inclined to the proximal side with respect to the body 38. The side port 40 may be inclined to the distal side with respect to the body 38. The side port 40 may protrude in a direction perpendicular to the axis of the body 38. The side port 40 may be configured to serve as a component separated from the body 38, and may be attached to the body 38.

The seal member 42 is held by the body 38. Specifically, an annular seal holding groove 48 is disposed in the distal portion of the body 38, and the seal member 42 is mounted on the seal holding groove 48. The seal member 42 is located inside the connection-purpose engagement portion 46, and is located on the distal side from the outlet 44b of the injection path 44. In a state where the air removal mechanism 16 is connected to the sheath hub 20, the seal member 42 is located on an outer periphery of the valve body 32.

In the state where the air removal mechanism 16 is connected to the sheath hub 20, the seal member 42 closely adheres to a proximal surface 20b of the sheath hub 20 (proximal surface 34c of the fixing member 34). In this case, a portion of the body 38 which configures the inside of the seal holding groove 48 comes close to or is attached to the proximal surface of the sheath hub 20.

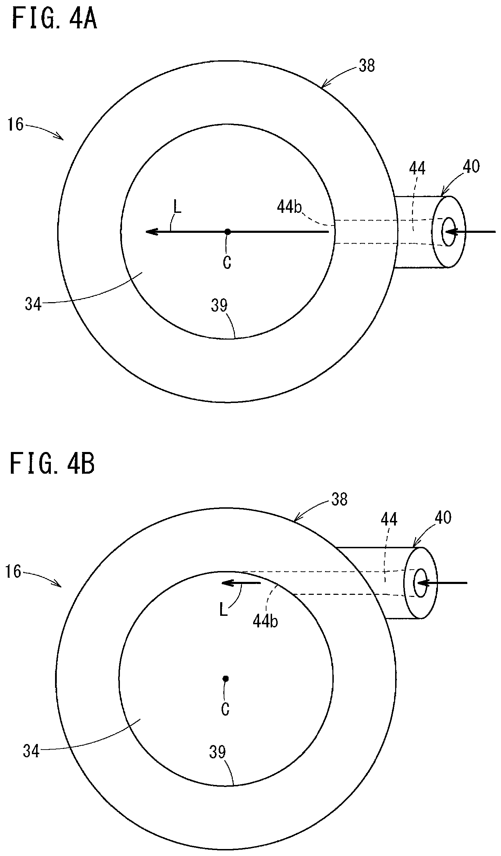

As illustrated in Fig. 4A, an illustrative example not falling under the scope of the claims, the outlet 44b of the injection path 44 is formed so that the liquid L reaches the outer peripheral side of the valve body 32 after passing above the axis (centerline C) of the valve body 32 when viewed in the axial direction of the valve body 32, when the liquid L flows out of the outlet 44b. As illustrated in Fig. 4B, the outlet 44b of the injection path 44 is formed so that the liquid L reaches the valve body 32 in the tangential direction of the valve body 32 when viewed in the axial direction of the valve body 32, when the liquid L flows out of the outlet 44b.

Next, an operation of the introducer 10 configured as described above will be described.

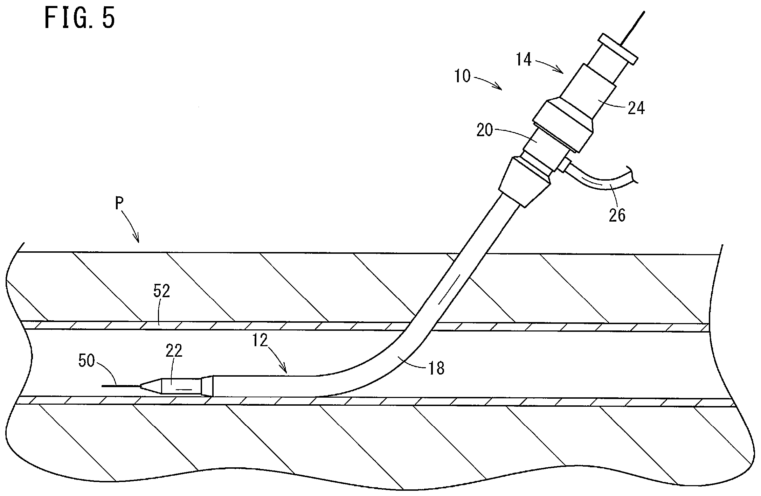

When the introducer 10 is used, as illustrated in Fig. 2, the dilator 14 is first inserted into the sheath 12, and the introducer 10 is brought into an assembled state. As illustrated in Fig. 5, the sheath 12 and the dilator 14 are inserted into a blood vessel 52 of a patient P along a guide wire 50. Specifically, the guide wire 50 is first inserted into the blood vessel 52 in advance by using a wire introduction needle, for example. Then, the guide wire 50 is inserted from the distal end of the dilator 14 combined with the sheath 12, and the sheath 12 and the dilator 14 are inserted into the blood vessel 52 along the guide wire 50.

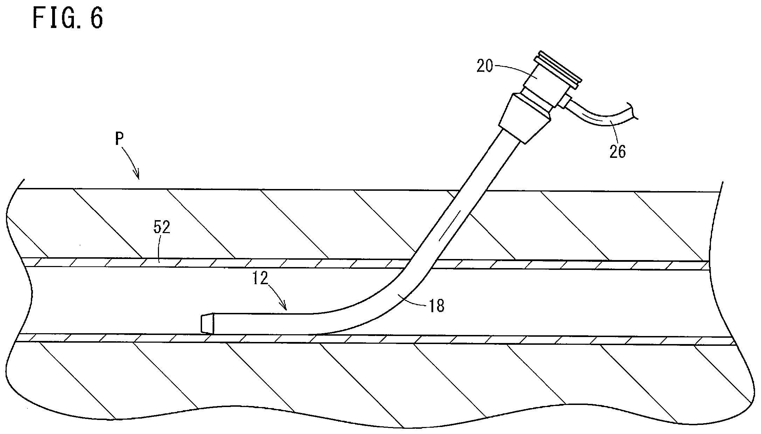

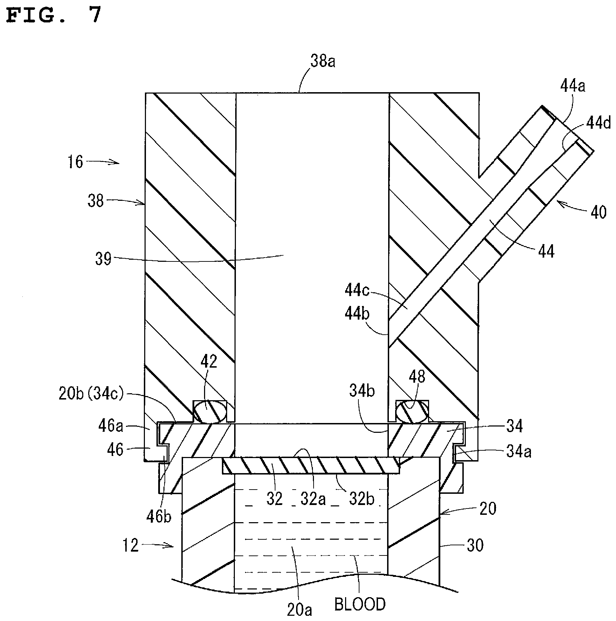

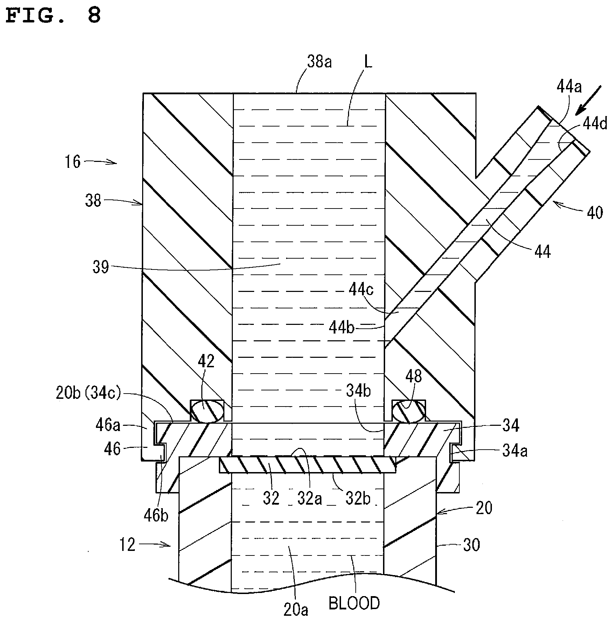

Next, as illustrated in Fig. 6, the guide wire 50 and the dilator 14 are removed from the sheath 12. Next, as illustrated in Fig. 7, the air removal mechanism 16 is connected to the sheath hub 20. In this manner, the hollow portion 39 of the air removal mechanism 16 is in a state of being located to face the valve body 32. Next, the proximal portion of the sheath hub 20 is brought into an upward facing state (state where a proximal opening 38a of the body 38 is caused to face upward). Then, in this state, as illustrated in Fig. 8, the liquid L (for example, a transparent liquid such as a physiological salt solution) is supplied to the hollow portion 39 via the injection path 44, and the hollow portion 39 is filled with the liquid L. In particular, if the transparent liquid is used as the liquid L, when another medical device 54 (for example, a catheter 54a) is inserted into the sheath 12 via the hollow portion 39 and the valve body 32 of the air removal mechanism 16, a user can easily recognize the center of the valve body 32.

In this case, the liquid L flowing out of the outlet 44b of the injection path 44 flows toward the valve body 32, and first directly collides with the valve body 32. Thereafter, the liquid L spreads over the valve body 32. Therefore, the valve body 32 side is first filled with the liquid L, and a liquid volume (liquid level) of the liquid L increases inside the hollow portion 39. In this manner, the air existing above the valve body 32 is extruded upward by the liquid L. That is, an upward region of the valve body 32 is filled with the liquid L, thereby removing the air existing above the valve body 32.

Next, as illustrated in Fig. 9, while a state where the proximal portion of the sheath hub 20 faces upward is maintained, another medical device 54 (for example, the catheter 54a) is inserted into the sheath 12 via the hollow portion 39 of the air removal mechanism 16. when another medical device 54 is inserted, the proximal portion of the sheath hub 20 may not face upward. Even in this case, the liquid L does not easily flow out of the hollow portion 39 due to surface tension. Then, after desired treatment is performed using the medical device 54, the medical device 54 is removed from the sheath 12. In this case, first, the proximal portion of the sheath hub 20 into which the medical device 54 is inserted is brought into the upward facing state. Next, the liquid L is supplied to the hollow portion 39 via the injection path 44, and the hollow portion 39 is filled with the liquid L again. Then, in this state, the medical device 54 is removed from the sheath 12. Furthermore, the liquid L is supplied to the hollow portion 39 via the injection path 44, and the hollow portion 39 is filled with the liquid L again. Thereafter, another medical device 54 is inserted into the sheath 12 via the hollow portion 39 of the air removal mechanism 16.

In this case, the air removal mechanism 16 according to the first embodiment achieves the following advantageous effects.

According to the air removal mechanism 16, if the liquid L is injected via the injection path 44 disposed in the body 38 into the hollow portion 39 of the air removal mechanism 16 located in the proximal portion (sheath hub 20) of the sheath 12 having the valve body 32 facing upward, the air existing above the valve body 32 (air inside the hollow portion 39) is extruded by the liquid L. Therefore, the air existing above the valve body 32 is removed. Therefore, when another medical device 54 such as the catheter 54a is inserted into the sheath 12, or when the medical device 54 is removed from the sheath 12, the air can be prevented from being mixed into the sheath 12.

In the injection path 44, at least the end portion flow path 44c on the outlet 44b side is inclined with respect to the axis of the body 38. In this manner, according to a simple configuration, the injection path 44 on which the outlet 44b is open toward the valve body 32 can be disposed inside the body 38.

The outlet 44b of the injection path 44 is open toward a position different from the center of the valve body 32. According to this configuration, the liquid L is likely to reach the outer peripheral side of the valve body 32. Therefore, the air existing above the valve body 32 can be effectively removed.

In Fig. 4B, the outlet 44b of the injection path 44 is formed so that the liquid L reaches the valve body 32 in the tangential direction of the valve body 32 when viewed in the axial direction of the valve body 32. According to this configuration, when the liquid L flowing out of the outlet 44b of the injection path 44 reaches the valve body 32, a swirling flow of the liquid L is formed above the valve body 32. Therefore, the air existing above the valve body 32 can be more effectively removed.

The air removal mechanism 16 includes the side port 40 which protrudes from the outer peripheral portion of the body 38, which has the inlet 44a of the injection path 44, and to which the liquid injection device can be connected. According to this configuration, the liquid injection device is connected to the side port 40, and the liquid L is discharged from the liquid injection device. In this manner, the liquid L can be easily injected to the hollowportion 39 via the injection path 44.

The side port 40 is inclined with respect to the axis of the body 38. According to this configuration, when in use, the side port 40 or the liquid injection device is less likely to cause trouble. In particular, the inlet (inlet 44a of the injection path 44) of the side port 40 is located at a position lower than that of the proximal opening 38a of the body 38. In this manner, when in use, the side port 40 or the liquid injection device is less likely to cause trouble. The inlet (inlet 44a of the injection path 44) of the side port 40 may be located at a position higher than that of the proximal opening 38a of the body 38. In this manner, the proximal opening 38a of the body 38 is likely to be filled with the liquid L.

The body 38 is configured to be separated from the sheath hub 20. According to this configuration, a configuration of the sheath 12 does not need to be greatly changed from the sheath in the related art, and the air can be prevented from being mixed into the sheath 12.

The body 38 is transparent. Accordingly, it is possible to visibly confirm a decrease in the liquid L or a stored liquid level inside the body 38 which is caused by the insertion and removal of the medical device 54. Therefore, it is possible to inject the liquid L from the injection path 44 before the liquid L existing above the valve body 32 runs short.

The flow path cross-sectional area of the injection path 44 is smaller than the flow path cross-sectional area of the hollow portion 39. According to this configuration, a flow velocity of the liquid L is allowed to increase. Therefore, the air inside the injection path 44 or the body 38 can be more effectively removed.

The air removal mechanism 16 is attached to the sheath hub 20 after the dilator 14 is removed from the sheath 12 inserted into the blood vessel 52. Therefore, the introducer 10 can be used in the same procedure as that of the introducer in the related art until the dilator 14 is removed from the sheath 12. Therefore, the introducer 10 can be easily used.

### [Second Embodiment]

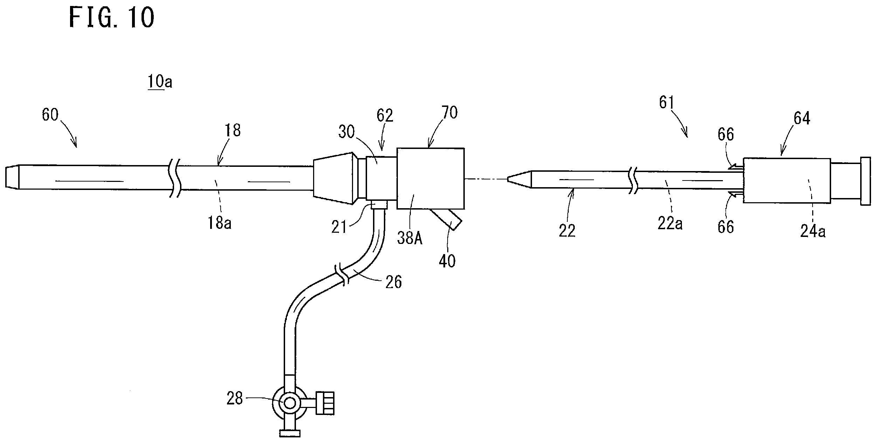

An introducer 10a illustrated in Fig. 10 includes an introducer sheath 60 (hereinafter, simply referred to as a "sheath 60") having a lumen 18a, and a dilator 61 used by being inserted into the lumen 18a of the sheath 60. The sheath 60 includes the elongated sheath shaft 18 configuring the sheath main body, and a sheath hub 62 connected to the proximal end of the sheath shaft 18. The dilator 61 has the elongated dilator shaft 22 configuring the dilator main body, and a dilator hub 64 connected to the proximal end of the dilator shaft 22.

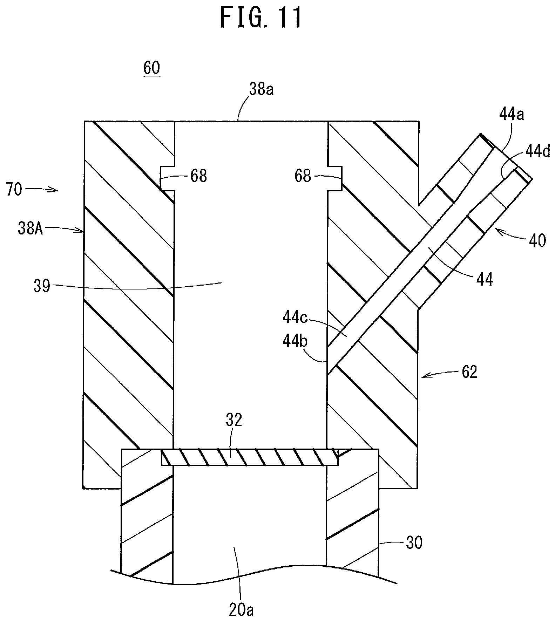

As illustrated in Fig. 11, the sheath hub 62 includes the housing 30 surrounding the lumen 20a, the valve body 32 located in the proximal portion of the housing 30, and an air removal mechanism 70 according to a second embodiment. The air removal mechanism 70 is fixed to the proximal portion of the housing 30. That is, the air removal mechanism 70 is integrally disposed in the sheath hub 62. The valve body 32 is fixed to the housing 30 by being interposed between the housing 30 and the air removal mechanism 70.

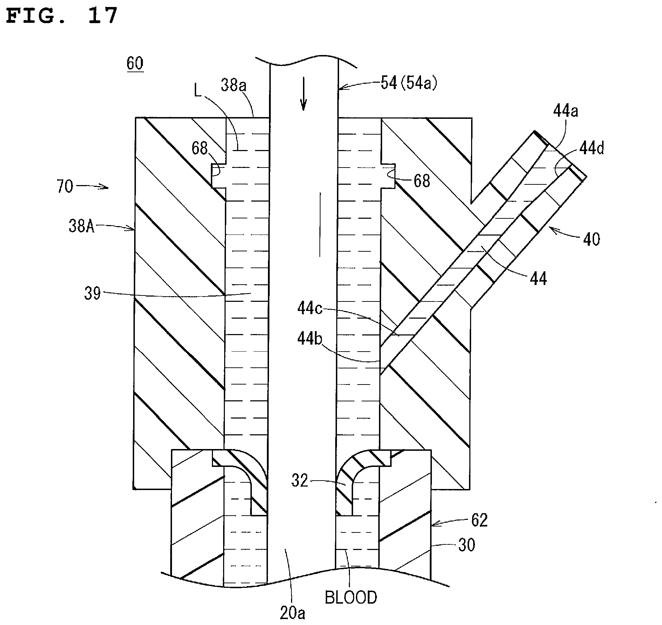

The air removal mechanism 70 includes a body 38A having the hollow portion 39 capable of storing the liquid L, and the side port 40 protruding from the outer peripheral portion of the body 38A. A configuration is adopted as follows. The medical device 54 (refer to Fig. 17) inserted into the sheath 60 can be inserted into the hollow portion 39, and the dilator shaft 22 can be inserted into the hollow portion 39. The body 38A has the injection path 44 communicating with the hollow portion 39 and having outlet 44b which is open toward the valve body 32.

In Fig. 10, the dilator hub 64 is configured to be attachable to and detachable from the air removal mechanism 70 (specifically, the body 38A). Therefore, the dilator hub 64 has an engagement claw 66 for engaging with the body 38A of the air removal mechanism 70. On the other hand, as illustrated in Fig. 11, the inner peripheral surface in the vicinity of the proximal end of the body 38A has an engagement groove 68 with which the engagement claw 66 of the dilator hub 64 can engage. The engagement groove 68 is disposed on the proximal side from the outlet 44b of the injection path 44 . An engagement mechanism between the body 38A and the dilator hub 64 may be disposed on the outer peripheral surface of the body 38A.

Next, an operation of the introducer 10a configured as described above will be described.

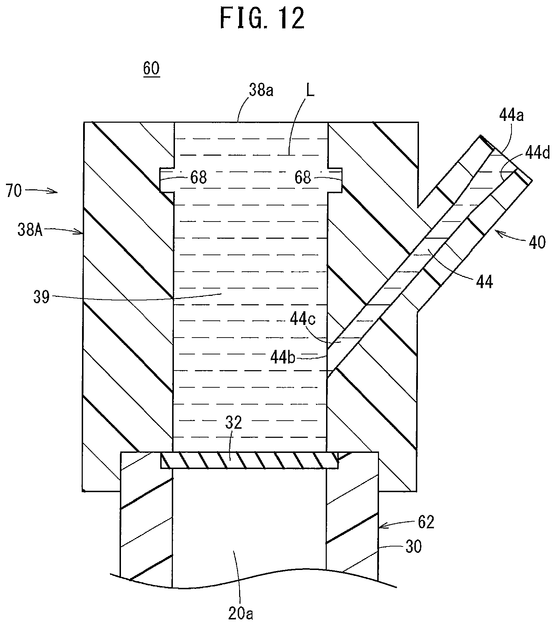

When the introducer 10a is used, as illustrated in Fig. 12, the proximal portion of the sheath hub 62 is first brought into an upward facing state (state where the proximal opening 38a of the body 38A is caused to face upward). Then, in this state, as illustrated in Fig. 12, the liquid L (for example, the physiological salt solution) is supplied to the hollow portion 39 via the injection path 44, and the hollow portion 39 is filled with the liquid L.

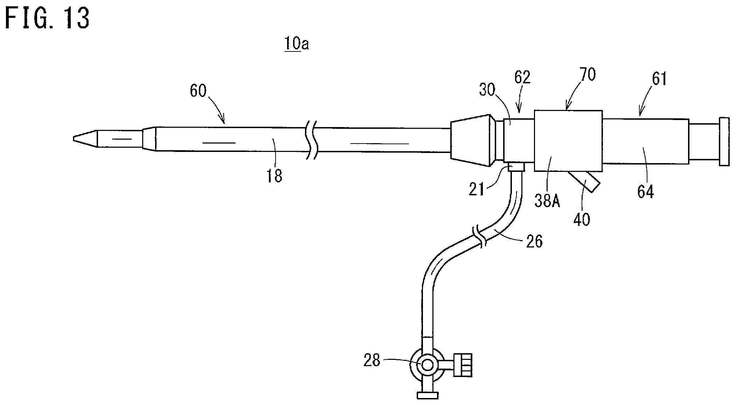

Next, as illustrated in Fig. 13, the dilator 61 is inserted into the sheath 60, and the introducer 10a is brought into an assembled state. In this case, the dilator shaft 22 is inserted into the housing 30 of the sheath hub 62 and the sheath shaft 18 via the hollow portion 39 (refer to Fig. 12) of the air removal mechanism 70 filled with the liquid L. In the assembled state of the introducer 10a, the distal portion of the dilator shaft 22 protrudes as much as a predetermined length from the distal opening of the sheath shaft 18. In addition, in the assembled state, the engagement claw 66 (refer to Fig. 10) disposed in the dilator hub 64 engages with the engagement groove 68 (refer to Fig. 11) disposed in the air removal mechanism 70.

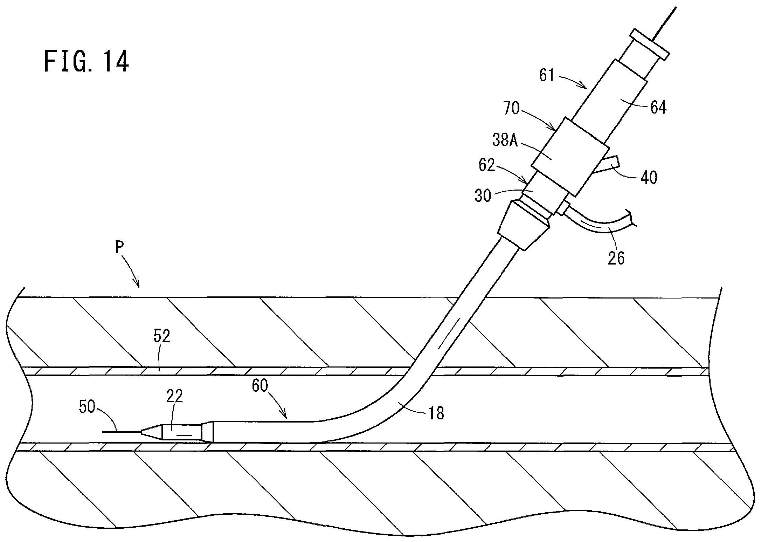

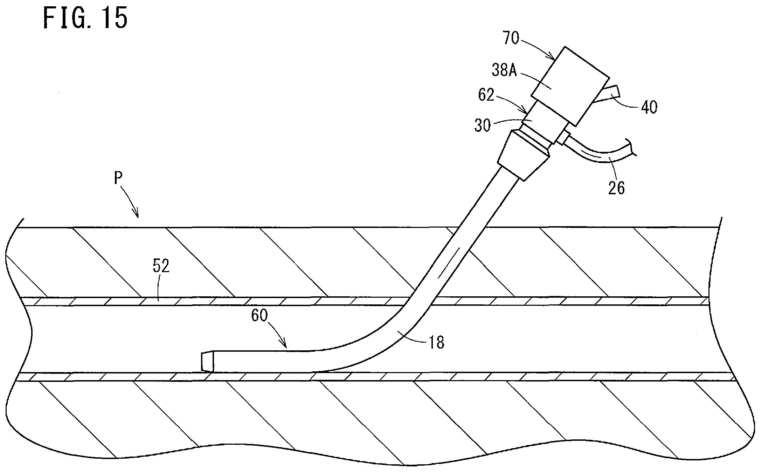

Next, as illustrated in Fig. 14, the sheath 60 and the dilator 61 are inserted into the blood vessel 52 along the guide wire 50. Next, as illustrated in Fig. 15, the guide wire 50 and the dilator 61 are removed from the sheath 60. The liquid L may be injected into the hollow portion 39 before the guide wire 50 and the dilator 61 are removed from the sheath 60.

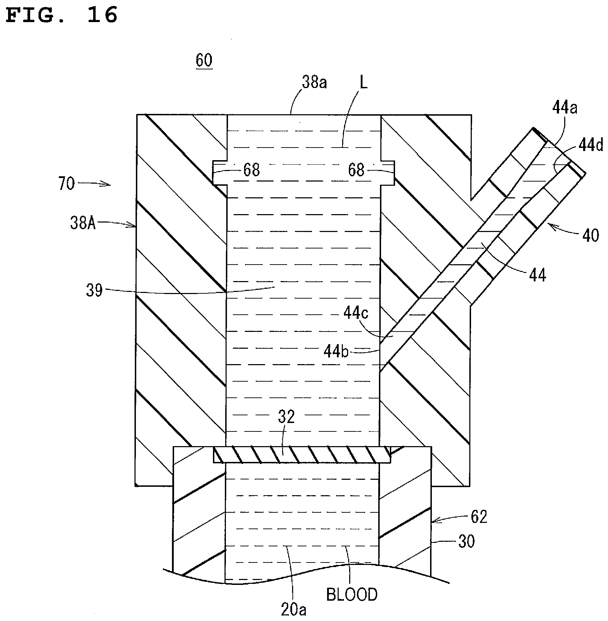

Next, the proximal portion of the sheath hub 62 is brought into an upward facing state (state where the proximal opening 38a of the body 38A is caused to face upward). Then, in this state, as illustrated in Fig. 16, the liquid L is supplied to the hollow portion 39 via the injection path 44, and the hollow portion 39 is filled with the liquid L.

Next, as illustrated in Fig. 17, while a state where the proximal portion of the sheath hub 62 faces upward is maintained, another medical device 54 (for example, the catheter 54a) is inserted into the sheath 60 via the hollow portion 39 of the air removal mechanism 70. Then, after desired treatment is performed using the medical device 54, the medical device 54 is removed from the sheath 60. In this case, first, the proximal portion of the sheath hub 62 into which the medical device 54 is inserted is brought into the upward facing state. Next, the liquid L is supplied to the hollow portion 39 via the injection path 44, and the hollow portion 39 is filled with the liquid L again. Then, in this state, the medical device 54 is removed from the sheath 60.

In this case, according to the second embodiment, the body 38A of the air removal mechanism 70 is integrally disposed in the sheath hub 62 (sheath proximal portion). According to this configuration, the hollow portion 39 of the air removal mechanism 70 is filled with the liquid L before the dilator 61 is inserted into the sheath 60. In this manner, when the dilator 61 is removed from the sheath 60, the air can be prevented from being mixed into the sheath 60. In addition, when the medical device 54 is inserted into the sheath 60, or when the medical device 54 is removed from the sheath 60, the air can be prevented from being mixed into the sheath 60.

With regard to the elements of the second embodiment which are common to the elements of the first embodiment, it is possible to achieve the same or similar operations and advantageous effects as those according to the first embodiment.

### [Third Embodiment]

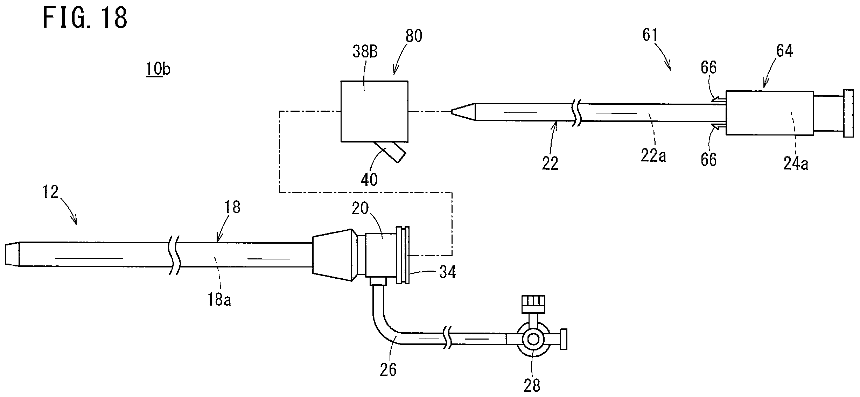

An introducer 10b illustrated in Fig. 18 includes the sheath 12 (same as the sheath 12 illustrated in Fig. 1), an air removal mechanism 80 according to a third embodiment which is used in combination with the sheath 12, and the dilator 61 (the same as the dilator 61 illustrated in Fig. 10) used by being inserted into the lumen 18a of the sheath 12 and the air removal mechanism 80.

In Fig. 18, the air removal mechanism 80 is configured to be mountable on the proximal portion of the sheath hub 20. According to the third embodiment, the air removal mechanism 80 is attachable to and detachable from the sheath hub 20 (removable after being mounted on the sheath hub 20). The air removal mechanism 80 may be configured so that the air removal mechanism 80 is not removable after being mounted once on the sheath hub 20.

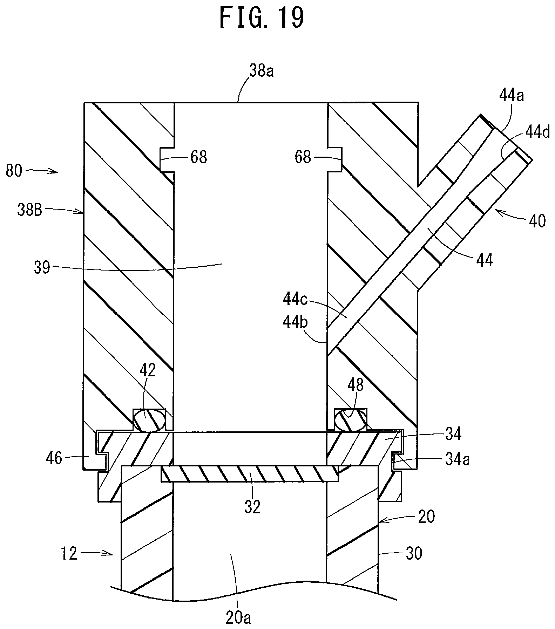

In Fig. 19, the air removal mechanism 80 is mounted on (connected to) the proximal portion of the sheath hub 20. The air removal mechanism 80 includes a body 38B having the hollow portion 39 capable of storing the liquid L and having the injection path 44, the side port 40 protruding from the outer peripheral portion of the body 38B, and the seal member 42 for sealing a portion between the body 38B and the sheath hub 20 in a liquid-tight manner. The body 38B is formed by adding the engagement groove 68 (same as the engagement groove 68 illustrated in Fig. 11) to the body 38 illustrated in Fig. 3. The dilator hub 64 has the engagement claw 66, and is configured to be attachable to and detachable from the air removal mechanism 80 (specifically, the body 38B). An engagement mechanism between the body 38B and the dilator hub 64 may be disposed on the outer peripheral surface of the body 38B.

Next, an operation of the introducer 10b configured as described above will be described.

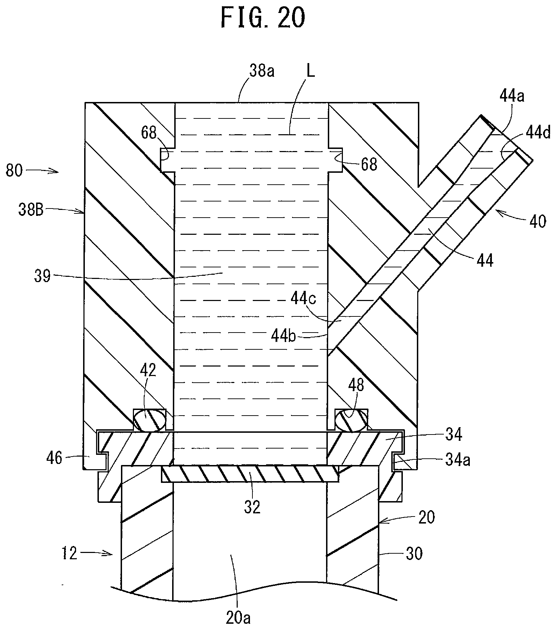

When the introducer 10b is used, as illustrated in Fig. 19, the air removal mechanism 80 is first connected to the proximal portion of the sheath hub 20. Next, the proximal portion of the sheath hub 20 is brought into an upward facing state (state where the proximal opening 38a of the body 38B is caused to face upward) . Then, in this state, as illustrated in Fig. 20, the liquid L (for example, the physiological salt solution) is supplied to the hollow portion 39 via the injection path 44, and the hollow portion 39 is filled with the liquid L.

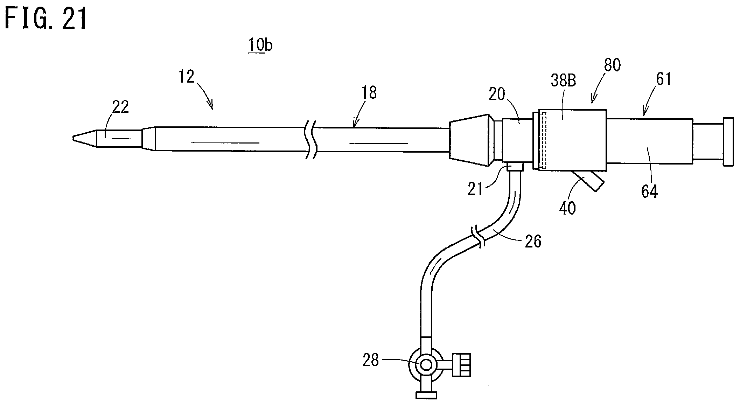

Next, as illustrated in Fig. 21, the dilator 61 is inserted into the sheath 12, and the introducer 10b is brought into an assembled state. In this case, the dilator shaft 22 is inserted into the housing 30 of the sheath hub 20 and the sheath shaft 18 via the hollow portion 39 (refer to Fig. 20) of the air removal mechanism 80 filled with the liquid L. In the assembled state of the introducer 10b, the distal portion of the dilator shaft 22 protrudes as much as a predetermined length from the distal opening of the sheath shaft 18. In addition, in the assembled state, the engagement claw 66 (refer to Fig. 18) disposed in the dilator hub 64 engages with the engagement groove 68 (refer to Fig. 19) disposed in the air removal mechanism 80.

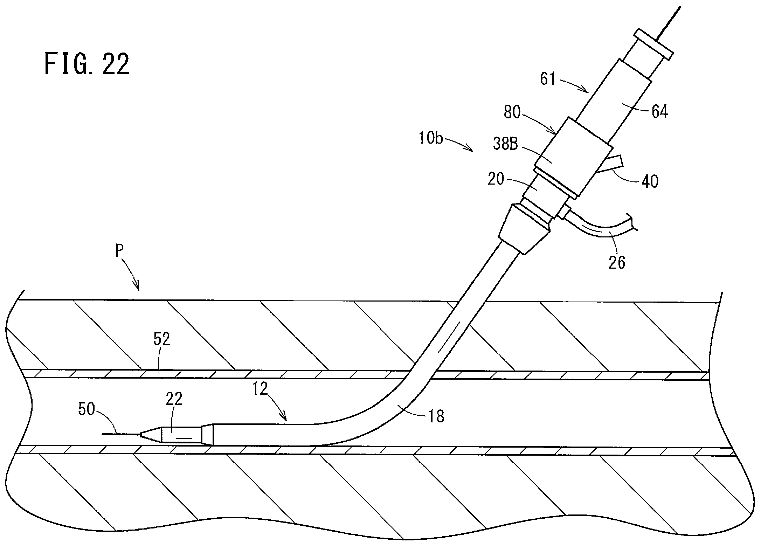

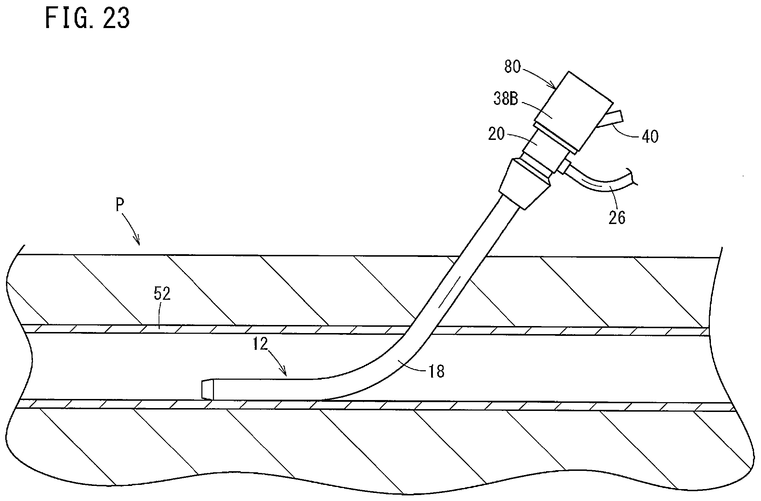

Next, as illustrated in Fig. 22, the sheath 12 and the dilator 61 are inserted into the blood vessel 52 along the guide wire 50. Next, as illustrated in Fig. 23, the guide wire 50 and the dilator 61 are removed from the sheath 12. The liquid L may be injected into the hollow portion 39 before the guide wire 50 and the dilator 61 are removed from the sheath 12.

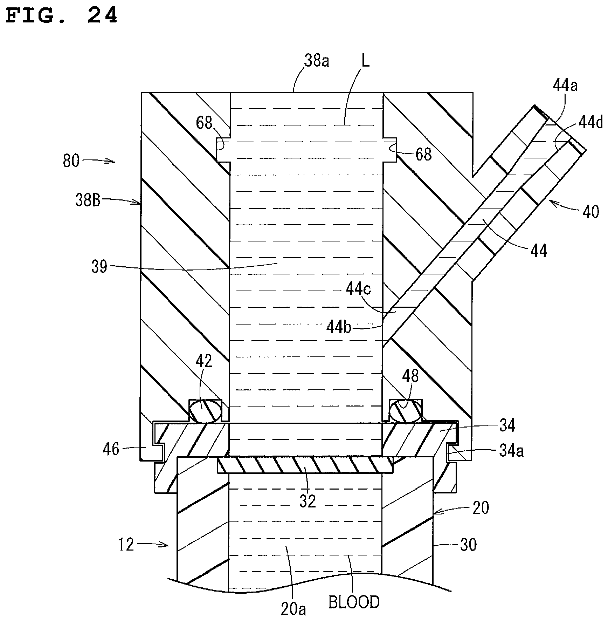

Next, the proximal portion of the sheath hub 20 is brought into an upward facing state (state where the proximal opening 38a of the body 38B is caused to face upward). Then, in this state, as illustrated in Fig. 24, the liquid L is supplied to the hollow portion 39 via the injection path 44, and the hollow portion 39 is filled with the liquid L.

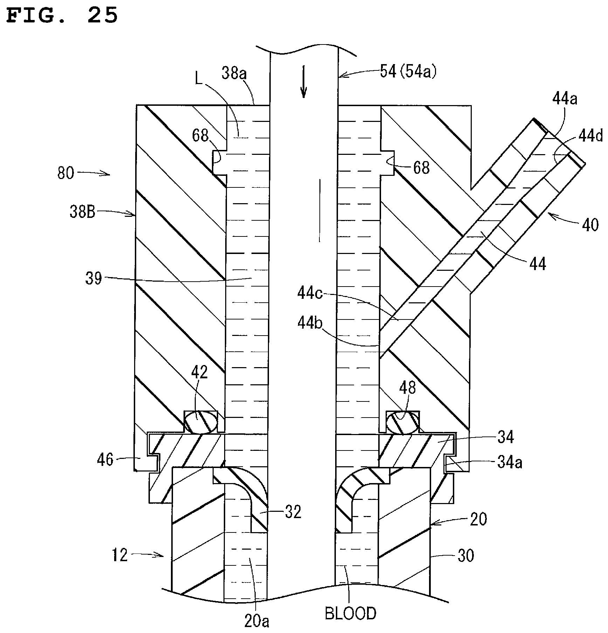

Next, as illustrated in Fig. 25, while a state where the proximal portion of the sheath hub 20 faces upward is maintained, another medical device 54 (for example, the catheter 54a) is inserted into the sheath 12 via the hollow portion 39 of the air removal mechanism 80. Then, after desired treatment is performed using the medical device 54, the medical device 54 is removed from the sheath 12. In this case, first, the proximal portion of the sheath hub 20 into which the medical device 54 is inserted is brought into the upward facing state. Next, the liquid L is supplied to the hollow portion 39 via the injection path 44, and the hollow portion 39 is filled with the liquid L again. Then, in this state, the medical device 54 is removed from the sheath 12.

In this case, according to the third embodiment, the air removal mechanism 80 is attachable to and detachable from the sheath hub 20, and the dilator 61 is configured to be attachable to and detachable from the air removal mechanism 80. According to this configuration, the hollow portion 39 of the air removal mechanism 80 mounted on the sheath hub 20 is filled with the liquid L before the dilator 61 is inserted into the sheath 12. In this manner, when the dilator 61 is removed from the sheath 12, the air can be prevented from being mixed into the sheath 12. In addition, when the medical device 54 is inserted into the sheath 12, or when the medical device 54 is removed from the sheath 12, the air can be prevented from being mixed into the sheath 12.

With regard to the elements of the third embodiment which are common to the elements of the first embodiment, it is possible to achieve the same or similar operations and advantageous effects as those according to the first embodiment.

### [Fourth Embodiment]

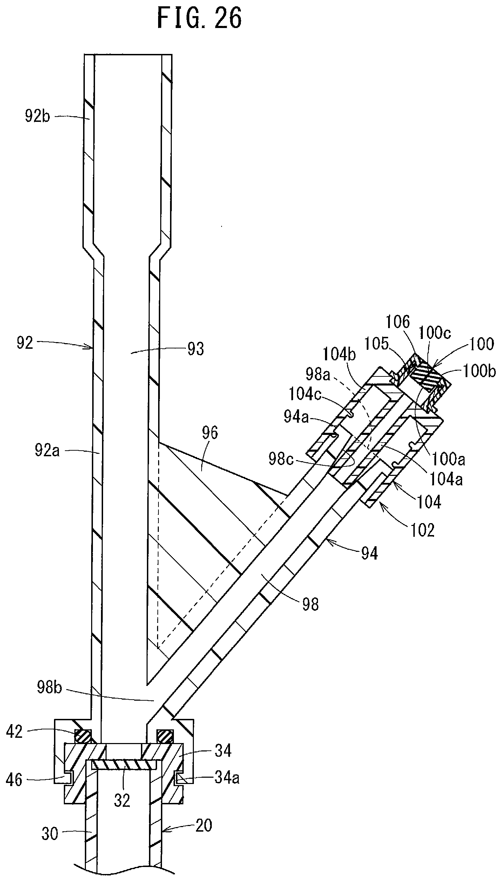

An air removal mechanism 90 according to a fourth embodiment illustrated in Fig. 26 is used in combination with the sheath 12, similarly to the air removal mechanism 16 (refer to Fig. 1) according to the first embodiment, and is attachable to and detachable from the sheath hub 20 (removable after being mounted on the sheath hub 20). The air removal mechanism 90 may be configured so that the air removal mechanism 90 is not removable after being mounted once on the sheath hub 20.

The air removal mechanism 90 includes a body 92 having a hollow portion 93 capable of storing the liquid L, a side port 94 protruding from the outer peripheral portion of the body 92, and the seal member 42 for sealing a portion between the body 92 and the sheath hub 20 in a liquid-tight manner. A reinforcement portion 96 is disposed between the body 92 and the side port 94. The reinforcement portion 96 is formed in a plate shape having a thickness smaller than the outer diameter (thickness) of the body 92 and the side port 94.

The body 92 is transparent. The outer diameter of the proximal portion 92b of the body 92 is larger than the outer diameter of the distal portion of the body 92. The distal portion of the body 92 has the connection-purpose engagement portion 46 which can engage with the sheath hub 20.

The hollow portion 93 is formed to face the valve body 32, and the medical device 54 (refer to Fig. 9) to be inserted into the sheath 12 is configured to be insertable. The hollow portion 93 is formed in a straight line shape along the axis of the sheath hub 20, and a cross-sectional shape perpendicular to the axis of the body 92 is a circular shape. The diameter of the proximal portion of the hollow portion 93 (outer diameter of a proximal portion 92b of the body 92) is larger than the diameter of the distal portion of the hollow portion 93 (outer diameter of a distal portion 92a of the body 92).

The body 92 has an injection path 98 communicating with the hollow portion 93 and having outlet 98b which is open toward the valve body 32. The flow path cross-sectional area of the injection path 98 is smaller than the flow path cross-sectional area of the hollow portion 93. An inlet 98a of the injection path 98 is open on a protruding side end surface of the side port 94. An outlet 98b of the injection path 98 is open on the inner peripheral surface of the body 92. The outlet 98b of the injection path 98 is open toward a position different from the center of the valve body 32 in the valve body 32. The inner peripheral portion of the protruding end (free end) of the side port 94 has a lure taper 98c.

The side port 94 is inclined to the proximal side with respect to the body 92. The sideport 94 has a valve body 100. Specifically, the air removal mechanism 90 includes a connector 102 having the valve body 100 which is attachable to and detachable from the side port 94. The protruding end of the side port 94 has a flange portion 94a protruding outward in the radial direction, and a female screw portion 102a disposed in the connector 102 can be screwed to the flange portion 94a.

In a case where the connector 102 is not provided, a distal nozzle 112 of a liquid injection device 110 can be fitted to the lure taper 98c of the side port 94. In a case where the connector 102 is not provided and the liquid injection device 110 has a lock portion provided with a female screw, the lock portion can be screwed to the flange portion 94a, and the distal nozzle 112 of the liquid injection device 110 can be fitted to the lure taper 98c.

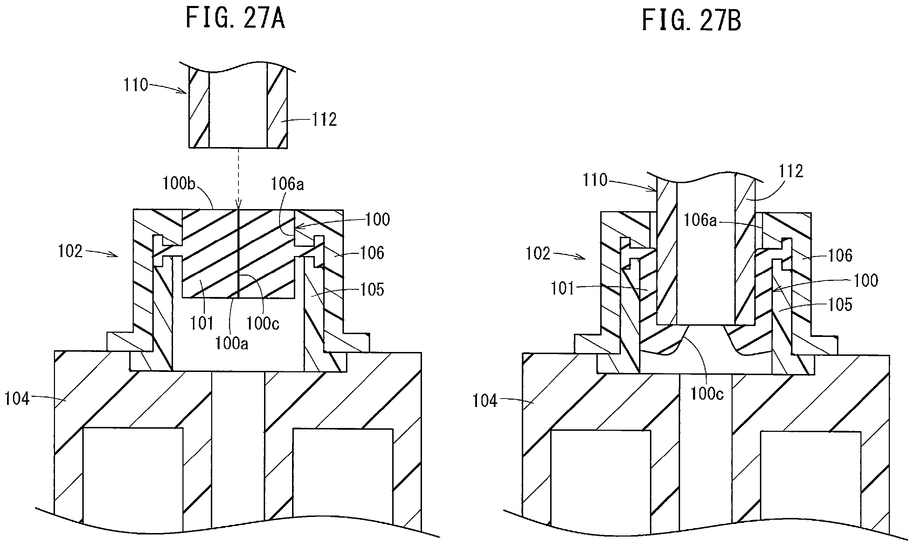

The connector 102 has a hollow connection cylinder 104, a support member 105 disposed in the proximal end of the connection cylinder 104, a valve body 100 supported by the support member 105, and a fitting member 106 which fixes the valve body 100 to the connection cylinder 104 and to which the distal nozzle 112 (refer to Fig. 27A) of the liquid injection device 110 can be fitted.

The connection cylinder 104 has a connection tube 104a which can be fitted to the lure taper 98c of the side port 94, and a lock portion 104b having a female screw 104c which can be screwed to the flange portion 94a of the side port 94. The valve body 100 of the connector 102 has a liquid-tight structure higher than that of the valve body 32 disposed inside the sheath proximal portion (sheath hub 20).

Specifically, the valve body 100 of the connector 102 is configured to have a smaller diameter than the valve body 32 of the sheath hub 20. The valve body 100 of the connector 102 is configured to be thicker than the valve body 32 of the sheath hub 20. The valve body 100 of the connector 102 has one slit 100c extending from a first end surface 100a to a second end surface 100b. The slit 100c may not be provided. In a state where the distal nozzle 112 of the liquid injection device 110 is not fitted to the fitting member 106, the slit 100c is closed, and a liquid-tight state is maintained.

In order to connect the liquid injection device 110 to the connector 102, as illustrated in Fig. 27A, the distal nozzle 112 of the liquid injection device 110 is caused to face the valve body 100 of the connector 102. Next, the distal nozzle 112 of the liquid injection device 110 is inserted into a hole portion 106a of the fitting member 106, and the valve body 100 (deformable portion 101) is pressed. As the distal side of the valve body 100 is pressed against the connection cylinder 104 side by the distal nozzle 112, the valve body 100 (deformable portion 101) is elastically deformed as illustrated in Fig. 27B, and the slit 100c is opened.

As described above, according to the fourth embodiment, the side port 94 has the valve body 100 having higher liquid-tightness than the valve body 32 disposed inside the sheath proximal portion (sheath hub 20). According to this configuration, when the liquid L is injected from the side port 94, the liquid injection device 110 can be fixed to the valve body 100 without any gap. When the liquid injection device 110 is separated from the side port 94, the valve body 100 disposed in the side port 94 has higher liquid-tightness than the valve body 32 disposed inside the sheath proximal portion (sheath hub 20). Therefore, the liquid-tightness can be maintained inside the side port 94. Therefore, the air can be prevented from being mixed into the air removal mechanism 90 (particularly, the side port 94).

As described above, the inner diameter and the outer diameter of the proximal portion 92b of the body 92 are respectively larger than the inner diameter and the outer diameter of the distal portion 92a of the body 92. The difference between the outer diameters of the proximal portion 92b and the distal portion 92a of the body 92 functions as a scale for measuring a liquid level. In this manner, if the liquid L reaches the distal portion 92a of the body 92, the distal portion 92a serves as a marker for injecting the liquid L. Therefore, it is possible to inject the liquid L from the injection path 98 before the liquid L existing above the valve body 32 runs short. In addition, in the body 92, the inner diameter of the proximal portion 92b is larger than the inner diameter of the distal portion 92a. Therefore, the medical device 54 is easily inserted.

As described above, the protruding end of the side port 94 has the flange portion 94a which can be screwed to the connector 102, and has the lure taper 98c. Therefore, the connector 102 or the liquid injection device 110 can be reliably fixed. Therefore, the air can be prevented from being mixed into the air removal mechanism 90 (particularly, the side port 94).

## Claims

1. An air removal mechanism (16, 70, 80, 90) configured to be attachable to and detachable from an introducer sheath (12, 60), said introducer sheath (12, 60) having a valve body (32, 100) disposed inside a sheath proximal portion,
the air removal mechanism (16, 70, 80, 90) comprising:
a body, said body having a hollow portion (39, 93) capable of storing a liquid (L), wherein the body of the air removal mechanism (16, 70, 80, 90) has an injection path (44, 98) communicating with the hollow portion (39, 93) and having an outlet (44b) which is open toward the valve body;
wherein the air removal mechanism (16, 70, 80, 90) is configured to allow, in a state where the body of the air removal mechanism (16, 70, 80, 90) is mounted on an introducer sheath (12, 60), a medical device (54) to be inserted into said introducer sheath (12, 60) via the hollow portion (39, 93) of the body of the air removal mechanism (16, 70, 80, 90),
**characterized in that**
the outlet (44b) of the injection path (44, 98) is open toward a position different from a center of the valve body (32).

2. The air removal mechanism (16, 70, 80, 90) according to claim 1, wherein
in the injection path (44, 98), at least an end portion flow path (44c) on the outlet side is inclined with respect to an axis of the body (38).

3. The air removal mechanism (16, 70, 80, 90) according to claim 1, wherein
the outlet (44b) of the injection path (44, 98) of the body of the air removal mechanism (16, 70, 80, 90) is formed so that the liquid (L) reaches the valve body (32, 100) in a tangential direction of the valve body (32) when viewed in an axial direction of the valve body (32).

4. The air removal mechanism (16, 70, 80, 90) according to any one of claims 1 to 3, further comprising:
a side port (40, 94) which protrudes from an outer peripheral portion of the body (38A, 38B), in which an inlet of the injection path (44, 98) is formed, and to which a liquid injection device (110) is connectable.

5. The air removal mechanism (16, 70, 80, 90) according to claim 4, wherein
the side port (40, 94) is inclined with respect to an axis of the body (38).

6. The air removal mechanism (16, 70, 80, 90) according to any one of claims 1 to 4, wherein
the body is configured to be separated from the sheath proximal portion.

7. The air removal mechanism (16, 70, 80, 90) according to claim 6, further comprising:
a seal member (42) held by the body and sealing a portion between the body and the sheath proximal portion in a liquid-tight manner.

8. The air removal mechanism (16, 70, 80, 90) according to any one of claims 1 to 6, wherein
the body is transparent.

9. The air removal mechanism (16, 70, 80, 90) according to claim 4, wherein
the side port (40, 94) is provided with another valve body (32, 100) having higher liquid-tightness than the valve body (32, 100) disposed inside the sheath proximal portion.

10. The air removal mechanism (16, 70, 80, 90) according to any one of claims 1 to 9, wherein
a flow path cross-sectional area of the injection path (44, 98) is smaller than a flow path cross-sectional area of the hollow portion (39, 93).

11. The air removal mechanism (16, 70, 80, 90) according to any one of claims 1 to 10, wherein
an inner diameter and an outer diameter of a proximal portion of the body are respectively larger than an inner diameter and an outer diameter of a distal portion of the body.

## Patentansprüche

1. Luftentfernungsmechanismus (16, 70, 80, 90), der konfiguriert ist, um an einer Einführungshülse (12, 60) anbringbar und von dieser abnehmbar zu sein, wobei die Einführungshülse (12, 60) einen Ventilkörper (32, 100) aufweist, der innerhalb eines proximalen Hülsenabschnitts angeordnet ist,
wobei der Luftentfernungsmechanismus (16, 70, 80, 90) Folgendes umfasst:
einen Körper, wobei der Körper einen hohlen Abschnitt (39, 93) aufweist, der in der Lage ist, eine Flüssigkeit (L) zu speichern, wobei der Körper des Luftentfernungsmechanismus (16, 70, 80, 90) einen Injektionsweg (44, 98) aufweist, der mit dem hohlen Abschnitt (39, 93) in Verbindung steht und einen Auslass (44b) aufweist, der zum Ventilkörper hin offen ist;
wobei der Luftentfernungsmechanismus (16, 70, 80, 90) konfiguriert ist, um in einem Zustand, in dem der Körper des Luftentfernungsmechanismus (16, 70, 80, 90) an einer Einführungshülse (12, 60) montiert ist, zu ermöglichen, dass eine medizinische Vorrichtung (54) über den hohlen Abschnitt (39, 93) des Körpers des Luftentfernungsmechanismus (16, 70, 80, 90) in die Einführungshülse (12, 60) eingeführt wird,
**dadurch gekennzeichnet, dass**
der Auslass (44b) des Injektionswegs (44, 98) zu einer Position hin offen ist, die sich von einer Mitte des Ventilkörpers (32) unterscheidet.

2. Luftentfernungsmechanismus (16, 70, 80, 90) nach Anspruch 1, wobei
im Injektionsweg (44, 98) mindestens ein Endabschnitt-Strömungsweg (44c) auf der Auslassseite in Bezug auf eine Achse des Körpers (38) geneigt ist.

3. Luftentfernungsmechanismus (16, 70, 80, 90) nach Anspruch 1, wobei
der Auslass (44b) des Injektionswegs (44, 98) des Körpers des Luftentfernungsmechanismus (16, 70, 80, 90) so ausgebildet ist, dass die Flüssigkeit (L) den Ventilkörper (32, 100) in einer tangentialen Richtung des Ventilkörpers (32) erreicht, wenn sie in einer axialen Richtung des Ventilkörpers (32) betrachtet wird.

4. Luftentfernungsmechanismus (16, 70, 80, 90) nach einem der Ansprüche 1 bis 3, ferner umfassend:
einen Seitenanschluss (40, 94), der von einem Außenumfangsabschnitt des Körpers (38A, 38B) vorsteht, in dem ein Einlass des Injektionswegs (44, 98) ausgebildet ist, und mit dem eine Flüssigkeitsinjektionsvorrichtung (110) verbindbar ist.

5. Luftentfernungsmechanismus (16, 70, 80, 90) nach Anspruch 4, wobei
der Seitenanschluss (40, 94) in Bezug auf eine Achse des Körpers (38) geneigt ist.

6. Luftentfernungsmechanismus (16, 70, 80, 90) nach einem der Ansprüche 1 bis 4, wobei
der Körper konfiguriert ist, um von dem proximalen Hülsenabschnitt getrennt zu werden.

7. Luftentfernungsmechanismus (16, 70, 80, 90) nach Anspruch 6, ferner umfassend:
ein Dichtungselement (42), das von dem Körper gehalten wird und einen Abschnitt zwischen dem Körper und dem proximalen Hülsenabschnitt in einer flüssigkeitsdichten Weise abdichtet.

8. Luftentfernungsmechanismus (16, 70, 80, 90) nach einem der Ansprüche 1 bis 6, wobei
der Körper transparent ist.

9. Luftentfernungsmechanismus (16, 70, 80, 90) nach Anspruch 4, wobei
der Seitenanschluss (40, 94) mit einem anderen Ventilkörper (32, 100) versehen ist, der eine höhere Flüssigkeitsdichtigkeit als der Ventilkörper (32, 100) aufweist, der innerhalb des proximalen Hülsenabschnitts angeordnet ist.

10. Luftentfernungsmechanismus (16, 70, 80, 90) nach einem der Ansprüche 1 bis 9, wobei
eine Strömungsweg-Querschnittsfläche des Injektionswegs (44, 98) kleiner als eine Strömungsweg-Querschnittsfläche des hohlen Abschnitts (39, 93) ist.

11. Luftentfernungsmechanismus (16, 70, 80, 90) nach einem der Ansprüche 1 bis 10, wobei
ein Innendurchmesser und ein Außendurchmesser eines proximalen Abschnitts des Körpers jeweils größer als ein Innendurchmesser und ein Außendurchmesser eines distalen Abschnitts des Körpers sind.

## Revendications

1. Mécanisme de purge d'air (16, 70, 80, 90) configuré pour pouvoir être attaché à et détaché d'une gaine d'introduction (12, 60), ladite gaine d'introduction (12, 60) ayant un corps de valve (32, 100) disposé à l'intérieur d'une partie proximale de gaine,
le mécanisme de purge d'air (16, 70, 80, 90) comprenant :
un corps, ledit corps ayant une partie creuse (39, 93) capable de stocker un liquide (L), dans lequel le corps du mécanisme de purge d'air (16, 70, 80, 90) a un trajet d'injection (44, 98) communiquant avec la partie creuse (39, 93) et ayant une sortie (44b) qui est ouverte vers le corps de valve ;
dans lequel le mécanisme de purge d'air (16, 70, 80, 90) est configuré pour permettre, dans un état où le corps du mécanisme de purge d'air (16, 70, 80, 90) est monté sur une gaine d'introduction (12, 60), à un dispositif médical (54) d'être inséré dans ladite gaine d'introduction (12, 60) via la partie creuse (39, 93) du corps du mécanisme de purge d'air (16, 70, 80, 90),
**caractérisé en ce que**
la sortie (44b) du trajet d'injection (44, 98) est ouverte vers une position différente d'un centre du corps de valve (32).

2. Mécanisme de purge d'air (16, 70, 80, 90) selon la revendication 1, dans lequel
dans le trajet d'injection (44, 98), au moins un trajet d'écoulement de partie d'extrémité (44c) sur le côté de sortie est incliné par rapport à un axe du corps (38).

3. Mécanisme de purge d'air (16, 70, 80, 90) selon la revendication 1, dans lequel
la sortie (44b) du trajet d'injection (44, 98) du corps du mécanisme de purge d'air (16, 70, 80, 90) est formée de sorte que le liquide (L) atteint le corps de valve (32, 100) dans une direction tangentielle du corps de valve (32) lorsqu'il est vu dans une direction axiale du corps de valve (32).

4. Mécanisme de purge d'air (16, 70, 80, 90) selon l'une quelconque des revendications 1 à 3, comprenant en outre :
un orifice latéral (40, 94) qui fait saillie d'une partie périphérique externe du corps (38A, 38B), dans lequel une entrée du trajet d'injection (44, 98) est formée, et auquel un dispositif d'injection de liquide (110) peut être connecté.

5. Mécanisme de purge d'air (16, 70, 80, 90) selon la revendication 4, dans lequel
l'orifice latéral (40, 94) est incliné par rapport à un axe du corps (38).

6. Mécanisme de purge d'air (16, 70, 80, 90) selon l'une quelconque des revendications 1 à 4, dans lequel
le corps est configuré pour être séparé de la partie proximale de gaine.

7. Mécanisme de purge d'air (16, 70, 80, 90) selon la revendication 6, comprenant en outre :
un élément d'étanchéité (42) maintenu par le corps et scellant une partie entre le corps et la partie proximale de gaine d'une manière étanche aux liquides.

8. Mécanisme de purge d'air (16, 70, 80, 90) selon l'une quelconque des revendications 1 à 6, dans lequel
le corps est transparent.

9. Mécanisme de purge d'air (16, 70, 80, 90) selon la revendication 4, dans lequel
l'orifice latéral (40, 94) est pourvu d'un autre corps de valve (32, 100) ayant une plus grande étanchéité aux liquides que le corps de valve (32, 100) disposé à l'intérieur de la partie proximale de gaine.

10. Mécanisme de purge d'air (16, 70, 80, 90) selon l'une quelconque des revendications 1 à 9, dans lequel
une section transversale de trajet d'écoulement du trajet d'injection (44, 98) est plus petite qu'une section transversale de trajet d'écoulement de la partie creuse (39, 93).

11. Mécanisme de purge d'air (16, 70, 80, 90) selon l'une quelconque des revendications 1 à 10, dans lequel
un diamètre intérieur et un diamètre extérieur d'une partie proximale du corps sont respectivement plus grands qu'un diamètre intérieur et un diamètre extérieur d'une partie distale du corps.
